# FASCICULE DE BREVET EUROPEEN

(11) **EP 4 277 643 B1**
(45) Date de publication et mention de la délivrance du brevet: **26.02.2025**
(21) Numéro de dépôt: 22700406.6
(22) Date de dépôt: 14.01.2022
(51) Int. Cl.: A61K 36/899, A61K 36/8968, A61P 17/16, A61K 9/00, A61P 37/08, A61K 47/36, A61K 47/44, A61K 9/06

(54) **COMPOSITION À BASE DE LIPIDES POLAIRES DE GERME DE BLÉ POUR LE TRAITEMENT DE LA DERMATITE ATOPIQUE**
ZUSAMMENSETZUNG AUF BASIS VON POLAREN WEIZENKEIMLIPIDEN ZUR BEHANDLUNG VON ATOPISCHER DERMATITIS
COMPOSITION BASED ON WHEAT GERM POLAR LIPIDS FOR THE TREATMENT OF ATOPIC DERMATITIS

(30) Priorité: 15.01.2021 FR 2100391
(43) Date de publication de la demande: 22.11.2023
(73) Titulaire: LABORATOIRES SVR, 91220 Le Plessis-Pâté (FR)
(72) Inventeur: TAMELGHAGHET, Myriam, 91220 Le Plessis-Pâté (FR)
(74) Mandataire: Cabinet Didier Martin
(86) Numéro de dépôt international: PCT/EP2022/050816
(87) Numéro de publication internationale: WO 2022/152893

(56) Documents cités:
- FR-A1- 2 779 646
- FR-B1- 2 886 846
- HO VALERIE ET AL: "Comparing the Potential for Irritation of a Ceramide-Based Moisturizer with a Urea-Based Moisturizer for Pediatric Atopic Dermatitis", DERMATOLOGY AND THERAPY, 1 August 2020 (2020-08-01), Switzerland, pages 807 - 813, XP055835296, Retrieved from the Internet <URL:https://www.ncbi.nlm.nih.gov/pmc/articles/PMC7367988/pdf/13555_2020_Article_388.pdf> [retrieved on 20210826], DOI: 10.1007/s13555-020-00388-6
- TESSEMA EFREM N. ET AL: "Potential Applications of Phyto-Derived Ceramides in Improving Epidermal Barrier Function", vol. 30, no. 3, 1 January 2017 (2017-01-01), CH, pages 115 - 138, XP055835318, ISSN: 1660-5527, Retrieved from the Internet <URL:https://www.karger.com/Article/PDF/464337> DOI: 10.1159/000464337
- KAHRAMAN EMINE ET AL: "Recent Advances on Topical Application of Ceramides to Restore Barrier Function of Skin", COSMETICS, vol. 6, no. 3, 20 August 2019 (2019-08-20), pages 52, XP055835308, DOI: 10.3390/cosmetics6030052
- DANBY SIMON G. ET AL: "An Investigation of the Skin Barrier Restoring Effects of a Cream and Lotion Containing Ceramides in a Multi-vesicular Emulsion in People with Dry, Eczema-Prone, Skin: The RESTORE Study Phase 1", vol. 10, no. 5, 1 October 2020 (2020-10-01), pages 1031 - 1041, XP055835303, ISSN: 2193-8210, Retrieved from the Internet <URL:https://link.springer.com/content/pdf/10.1007/s13555-020-00426-3.pdf> DOI: 10.1007/s13555-020-00426-3
- "CERAMOSIDES(TM) HP : ingrédient actif anti-âge, anti-vieillissement, anti-inflammatoire, anti-oxydant, agent d'amélioration du teint de la peau, et agent hydratant pour la préparation de formulations cosmétiques à usage topique ED - Darl Kuhn", IP.COM, IP.COM INC., WEST HENRIETTA, NY, US, 7 July 2017 (2017-07-07), XP013175336, ISSN: 1533-0001

## Description

La présente demande de brevet revendique la priorité de la demande de brevet Français FR 21/00391 déposée le 15/01/2021.

### Domaine de l'invention

La présente invention concerne une composition pour le traitement de la dermatite atopique et, plus spécifiquement, pour le traitement de la dermatite atopique consécutive d'une réaction allergique aux acariens de poussière chez un sujet.

### Etat de l'art

Les pathologies cutanées inflammatoires ou dermatoses inflammatoires sont des désordres cutanés accompagnés d'une composante inflammatoire. Ces maladies affectent la peau et les muqueuses et se caractérisent par des manifestations inesthétiques comme des rougeurs et/ou des plaques de desquamation. On peut citer, à titre d'exemples non limitatifs, la dermatite atopique, l'eczéma, le psoriasis, la rosacée, ou encore l'acné. Le plus souvent, ces pathologies résultent de désordres immunitaires associés aux phénomènes inflammatoires.

La dermatite atopique est la manifestation cutanée de l'atopie. C'est une dermatose inflammatoire chronique, survenant sur un terrain génétiquement déterminé. Elle touche 15 à 30% des enfants et 2 à 10% des adultes. Sa prévalence est en augmentation constante dans les pays industrialisés ; elle a doublé, voire triplé, au cours des trois dernières décennies et elle est maintenant considérée comme une préoccupation majeure de la santé publique. La dermatite atopique est souvent associée à d'autres désordres atopiques, tels que la rhinite allergique et l'asthme. Cette affection apparaît le plus souvent au cours de la petite enfance et se caractérise par des éruptions répétées pendant plusieurs années. Elle évolue par poussées entrecoupées de rémissions spontanées. Les lésions se caractérisent par une sécheresse cutanée importante associée à des manifestations inflammatoires : éruptions érythémateuses papuleuses, vésiculeuses, squameuses et très prurigineuses. Cette pathologie est caractérisée par une réponse inappropriée du système immunitaire dirigée contre des antigènes de l'environnement, appelés allergènes. Dans la majorité des cas, les acariens sont à l'origine de la réaction allergique.

Les acariens sont un taxon des arachnides qui comptent près de 50 000 espèces répertoriées. Parmi celles-ci, les acariens de poussière, *Dermatophagoides pteronyssinus* ou *Dermatophagoides farinae,* sont des espèces d'acariens domestiques. Ils se nourrissent de détritus organiques tels que la peau morte et s'épanouissent dans un environnement stable comme les logements. L'intestin de l'acarien contient des enzymes digestives puissantes (notamment les protéases) qui persistent dans les excréments et sont des inducteurs majeurs de réactions allergiques telle que la respiration sifflante. Parmi ces enzymes, on peut citer la peptidase 1 qui est une protéase à cystéine et constitue certainement l'un des allergènes majeurs des acariens de poussière. Les variants de celle-ci comprennent Der p1 qui est la peptidase 1 de l'espèce *Dermatophagoides pteronyssinus* ou Der f1 qui est la peptidase 1 de l'espèce *Dermatophagoides farinae.* L'activité de la peptidase 1 engendre une perturbation de l'épithélium respiratoire, de l'épithélium intestinal mais également des jonctions serrées de la peau.

Considérant la complexité de la dermatite atopique, ainsi que les effets secondaires associés aux traitements disponibles pour cette pathologie, il existe un besoin de disposer de nouvelles compositions utiles pour le traitement et/ou la prévention de cette pathologie.

### Description détaillée de l'invention:

Les inventeurs ont maintenant mis en évidence qu'un extrait à base de lipides polaires de germe de blé, lequel extrait est riche en céramides et en digalactosyldiacylglycérides (DGDG), permettait d'inhiber l'effet des acariens de la poussière sur la e-cadhérine *in vitro* et *ex-vivo,* alors que des céramides seules issus d'autres sources ne présentent aucun effet.

Les céramides sont des sphingolipides résultant de la combinaison d'un acide gras avec la sphingosine via une liaison amide. Ces molécules sont présentes en abondance dans les membranes cellulaires, où elles servent à la constitution des sphingomyélines. Il est à noter que les céramides ne jouent pas qu'un rôle structurel dans les membranes biologiques, puisqu'ils peuvent également revêtir des fonctions de signalisation lipidique. Parmi ces fonctions, on peut citer des actions sur la différenciation cellulaire, la mort cellulaire programmée (apoptose) ou encore la prolifération cellulaire.

Les galactoglycérolipides (i.e. monogalactosyldiacylglycérol, MGDG et digalactosyldiacylglycérides, DGDG) sont les lipides majeurs des membranes des chloroplastes, représentant jusqu'à 80% de la composition membranaire plastidiale.

De surcroît, les inventeurs ont mis en évidence qu'une combinaison d'une telle huile extraite de la farine de blé avec des oligofructanes d*'Ophiopogon japonicus* permettait d'obtenir un effet synergique sur la dermatite atopique.

En conséquence, un premier objet porte sur une composition dermatologique comprenant au moins un extrait à base de lipides polaires de germe de blé pour une utilisation pour prévenir et/traiter une dermatite atopique consécutive d'une réaction allergique aux acariens de poussière chez un sujet.

Avantageusement, pour une utilisation pour prévenir et/ou traiter l'altération des j onctions serrées associée à une dermatite atopique consécutive d'une réaction allergique aux acariens de poussière chez un sujet.

Pour ce qui est du sujet, il s'agit d'un mammifère et, de préférence, d'un humain.

La composition selon l'invention est appliquée sur la surface de peau affectée au moins une fois par jour.

- La composition selon l'invention est appliquée pendant au moins une semaine pour obtenir la meilleure efficacité et, de préférence, pendant au moins un mois.

Selon un mode de réalisation préférée, la composition selon l'invention vise à prévenir et/ou à traiter à une dermatite atopique consécutive d'une réaction allergique à la peptidase 1 (Der p1 ou Der f1) des acariens de poussière chez un sujet.

L'extrait à base de lipides polaires de germe de blé (ou de farine de blé) peut se trouver sous forme d'une huile, d'une émulsion, ou d'une poudre.

Dans le cas d'une poudre, de tels extraits sont disponibles chez HITEX (LIPOWHEAT POWDER), ENNAGRAM (CENNAMIDE poudre dispersible dans l'eau), E.P.I France (WHEAT CERASOMES POWDER) et chez SEPPIC (CERAMOSIDES poudre).

Dans le cadre d'une huile, de tels extraits correspondent simplement à une huile de germe de blé. Maintenant, de tels extraits sont disponibles chez HITEX (LIPOWHEAT OIL), ENNAGRAM (CENNAMIDE « wheat lipids at 15% CERs »), E.P.I France (WHEAT CERASOMES OIL) et chez SEPPIC (CERAMOSIDES huile).

Dans le cadre d'une émulsion, là encore il s'agit simplement d'une huile de germe de blé émulsionnée. Maintenant, de tels extraits sont disponibles chez ENNAGRAM (CENNAMIDE « water soluble émulsion at 5% CERs ») et chez E.P.I France (WHEAT CERASOMES EMULSION) et chez SEPPIC (CERAMOSIDES huile).

De préférence, l'extrait est sous forme de poudre.

Une telle poudre comprend alors près de 50% de céramides (glycosphingolipides et glycolipides) et près de 40% de digalactosyldiacylglycéri des.

Avantageusement, la poudre utilisée présente une teneur en céramides de 40% ou plus (en poids par rapport au poids total de l'huile), de préférence de 50% ou plus.

Avantageusement encore, la poudre utilisée présente une teneur en digalactosyldiacylglycérides de 30% ou plus (en poids par rapport au poids total de l'huile), de préférence de 40% ou plus.

Des méthodes d'obtention d'un tel extrait, qu'il soit sous la forme d'une huile, d'une émulsion ou d'une poudre sont connues de l'homme du métier.

Des méthodes d'extraction sont notamment dans les brevet FR 2 886 846 B1 ou FR 2 779 646.

De façon synthétique, l'huile correspond à la fraction huileuse totale de germes de blé (sous forme de farine préférentiellement pour en faciliter l'extraction) qui est extraite en utilisant des solvants aptes à solubiliser les lipides. Un tel solvant est typiquement un alcool tel que le méthanol, l'isopropanol, le 1-butanol ou l'éthanol. Une telle extraction s'opère typiquement sous agitation et, en utilisant avantageusement l'effet bénéfique de la température, dont la valeur est choisie en fonction du solvant utilisé. Le résultat de cette étape d'extraction est un mélange hétérogène constitué du solide épuisé des lipides et la solution d'extraction. Il importe donc de collecter la phase liquide en séparant la phase solide de celle-ci (criblage, décantation, filtration, etc.). Cette séparation peut se faire avec l'adjonction préalable d'un adjuvant capable de fixer l'eau et de retenir les protéines tel que la terre de diatomée.

L'huile peut être obtenue simplement par distillation du filtrat obtenu. Cette huile de farine (germe) de blé comprend alors en général de l'ordre de 15% de céramides et de 15% de digalactosyldiacylglycérides. Dans le cadre d'une émulsion, cette huile est simplement émulsionnée selon des techniques bien connues de l'homme du métier et la proportion de céramides et de digalactosyldiacylglycérides descend alors à environ 5% (du fait de l'adjonction d'eau et d'émulsionnants entraînant leur dilution).

Maintenant, et dans le cas où l'on souhaiterait une poudre correspondant à un extrait de lipides polaires « enrichi », il convient simplement d'utiliser la différence de solubilité de ces lipides polaires par rapport aux lipides apolaires. Pour se faire, il est possible de précipiter directement les lipides polaires en additionnant à l'huile un solvant dans lequel ils ne sont pas solubles (ex. acétone) comme décrit dans le brevet FR 2 779 646 B1. Il est également possible, et en partant du filtrat (et non du distillat qui constitue l'huile), d'opérer une extraction liquide/liquide en utilisant un système biphasique avec deux solvants non miscibles dont l'un ne permet pas de solubiliser les lipides polaires (ex. heptane/méthanol) comme décrit dans le brevet FR 2 886 846 B1. Les lipides polaires se maintenant préférentiellement dans l'une des phases et des extractions successives permettent ainsi un enrichissement en lipides polaires avant d'opérer une évaporation du solvant.

Avantageusement, la teneur de la composition selon l'invention en extrait, que ce soit sous forme d'huile, d'émulsion ou de poudre, est comprise entre 0,01 et15% (en poids par rapport au poids total de la composition).

Dans le cas d'une poudre, la teneur est de préférence elle est comprise entre 0,1 et 0,5%.

La composition selon l'invention est destinée à une application topique sur la peau, notamment la peau du visage et du corps. A ce titre, la composition selon l'invention prend la forme d'une crème, d'un sérum, d'un lait, d'une lotion ou d'un gel.

De préférence, la composition selon l'invention prendra la forme d'une crème.

Selon un autre mode de réalisation préférée, la composition selon l'invention comprend en outre au moins des oligofructanes d*'Ophiopogon japonicus.*

*Ophiopogon japonicus* ou muguet du japon, est une plante herbacée vivace que l'on trouve à l'état naturel en Chine, au Japon, en Inde ou encore au Vietnam.

Par oligofructanes d*'Ophiopogon japonicus,* on entend les polysaccharides présents dans les tubercules, lesquels sont majoritairement des fructosanes.

Avantageusement, la teneur de la composition selon l'invention oligofructanes d' *Ophiopogonjaponicus* est comprise entre 0,01 et 5% (en poids par rapport au poids total de la composition), de préférence elle est comprise entre 0,05 et 1% et, de manière particulièrement préférée, entre 0,3 et 0,7%.

De façon générale, la composition selon l'invention pourra comprendre de nombreux types d'adjuvants ou de principes actifs utilisés dans les formulations pharmaceutiques ou cosmétiques, de préférence dermatologiques, qu'il s'agisse de corps gras, de solvants organiques, d'épaississants, de gélifiants, d'adoucissants, d'antioxydants, d'opacifiants, de stabilisants, de tensioactifs moussants et/ou détergents, d'émollients, de surgraissants, de parfums, d'émulsionnants ioniques ou non, de charges, de séquestrants, de chélateurs, de conservateurs, d'huiles essentielles, de matières colorantes, de pigments, d'actifs hydrophiles ou lipophiles, d'humectants, comme par exemple la glycérine ou les glycols, de conservateurs, de colorants, d'actifs cosmétiques, de filtres solaires minéraux et/ou organiques, de charges minérales, de charges synthétiques, d'élastomères siliconés, ou d'extraits de plantes ou encore de vésicules lipidiques, ou bien tout autre ingrédient habituellement utilisé en cosmétique.

Comme exemples d'huiles, on peut citer les paraffines, les isoparaffines, les huiles blanches minérales, les huiles végétales (provenant de fleurs, de fruits, de légumes, d'arbres, de céréales, d'oléagineux ...), les huiles animales, les huiles de synthèse, les huiles siliconées et les huiles fluorées ; et plus particulièrement : les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sisymbre officinale, l'huile d'avocat, l'huile de calendula, les huiles issues de fleurs ou de légumes; les huiles végétales éthoxylées ; les huiles d'origine animale, telles que le squalène, le squalane ; les huiles minérales, telles que l'huile de paraffine, l'huile de vaseline et les isoparaffines ; les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylène glycol ; les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadécane, l'isododécane, les huiles perfluorées et les huiles de silicone. Parmi ces dernières, on peut plus particulièrement citer les diméthylpolysiloxanes, méthylphénylpolysiloxanes, les silicones modifiées par des aminés, les silicones modifiées par des acides gras, les silicones modifiées par des alcools, les silicones modifiées par des alcools et des acides gras, des silicones modifiées par des groupements polyéther, des silicones époxy modifiées, des silicones modifiées par des groupements fluorés, des silicones cycliques et des silicones modifiées par des groupements alkyles.

Comme autre matière grasse, on peut citer les alcools gras, linéaires ou ramifiés, saturés ou insaturés, les mélanges d'alcools gras, linéaires et/ou ramifiés, saturés et/ou insaturés, ou les acides gras, linéaires ou ramifiés, saturés ou insaturés, des mélanges d'acides gras linéaires ou ramifiés, saturés ou insaturés.

Parmi les polymères épaississants et/ou émulsionnant utilisables, il y a par exemple, les homopolymères ou copolymères de l'acide acrylique ou de dérivés de l'acide acrylique, les homopolymères ou copolymères de l'acide méthacrylique ou dérivés de l'acide méthacrylique, les homopolymères ou copolymères de l'acrylamide, les homopolymères ou copolymères de dérivés de l'acrylamide, les homopolymères ou copolymères de l'acide acrylamidométhyl propanesulfonique, les homopolymères ou copolymères de monomères vinyliques, les homopolymères ou copolymères de chlorure de triméthylaminoéthylacrylate, les hydrocolloïdes d'origine végétale ou bio synthétique comme par exemple la gomme de xanthane, la gomme de karaya, les carraghénates, les alginates ; les silicates ; la cellulose et ses dérivés ; l'amidon et ses dérivés hydrophiles; les polyuréthanes.

Parmi les polymères de type polyélectrolytes pouvant être mis enjeu dans la production d'une phase aqueuse gélifiée apte à être utilisée dans la préparation d'émulsions E/H, H/E, E/H/E ou H/E/H, ou d'un gel aqueux comprenant le SEPIBIO^{™} POTENTILLA 217, il y a par exemple, les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'AMPS et du N,N-diméthylacrylamide., les terpolymères de l'AMPS, de l'acide acrylique et du N,N-diméthylacrylamide, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone.

Parmi les cires utilisables dans les compositions selon l'invention, on peut citer par exemple la cire d'abeille, la cire de carnauba, la cire de Candelilla, la cire d'Ouricoury, la cire du Japon, la cire de Chine, la cire de son de riz, la cire de Montan, la cire de fibre de liège ou de canne à sucre, les cires de paraffines, les cires de lignite, les cires microcristallines, la cire de lanoline, l'ozokérite, la cire de polyéthylène, les huiles hydrogénées, les cires de silicone, les cires d'alcénones, les cires végétales, les alcools gras et les acides gras solides à température ambiante, les glycérides solides à température ambiante.

Parmi les émulsionnants utilisables dans les compositions selon l'invention, on peut citer :
- les esters gras d'alkylpolyglycosides éventuellement alcoxylés ;
- les esters gras alcoxylés ;
- les carbamates de polyalkylène glycols à chaînes grasses ;
- les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras ;
- les associations de tensioactifs émulsionnants choisis parmi les alkylpolyglycosides ;
- les associations d'alkylpolyglycosides et d'alcools gras, les esters de polyglycérols ou de polyglycols ou de polyols.

Parmi les tensioactifs utilisables dans les compositions selon l'invention, on peut citer: les tensioactifs anioniques, cationiques, amphotères ou non ioniques topiquement acceptables habituellement utilisés dans ce domaine d'activité.

Parmi les tensioactifs anioniques utilisables dans les compositions selon l'invention, on citera particulièrement les sels de métaux alcalins, les sels de métaux alcalino-terreux, les sels d'ammonium, les sels d'acides aminés, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates.

Parmi les tensioactifs amphotères utilisables dans les compositions selon l'invention, on pourra citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Afin de potentialiser encore l'activité obtenue par la composition selon l'invention, celle-ci pourra être associée avec d'autres actifs notamment ceux connus pour leur action anti-âge, raffermissante, restructurante, stimulante, énergisante, anti-ride, décontractante, hydratante, antimicrobienne, séborrégulatrice, purifiante, apaisante, relaxante, décontractante, anti-stress, éclaircissante, immunomodulatrice, stimulatrice du renouvellement cellulaire, liftante, repulpante, amélioratrice de l'éclat du teint, etc.

Selon un deuxième objet, la composition décrite précédemment est utilisée dans une méthode qui vise à prévenir et/ou à traiter une dermatite atopique consécutive d'une réaction allergique aux acariens de poussière chez un sujet et comprend une étape d'administration d'une quantité thérapeutiquement efficace de cette dernière sur une surface de peau dudit sujet.

Par « quantité thérapeutiquement efficace », on entend une quantité suffisante pour aboutir à l'effet biologique désiré.

Avantageusement, la méthode selon l'invention vise à prévenir et/ou à traiter à une dermatite atopique consécutive d'une réaction allergique à la peptidase 1 (Der p1 ou Der f1) des acariens de poussière chez un sujet.

L'invention sera mieux comprise à la lumière des exemples suivants, qui ne sont donnés qu'à titre purement illustratif et n'ont pas pour but de limiter la portée de l'invention, définie par les revendications annexées.

### Exemples

### Exemple 1 : Préparation d'une composition selon l'invention :

Pour cette étude *in vitro,* 3 céramides nommées céramides HP (SEPPIC, poudre), les céramides Y30 (DOOSAN) et DS (DOOSAN) ont été testés.

A cette fin, des kératinocytes extraits de prépuce ont été mis en culture soit dans le milieu de culture de kératinocytes KGM (LONZA) selon les conditions définies par le fabricant. Ces kératinocytes ont été cultivés en présence ou en absence d'extraits d'acariens de la poussière (HDM) à 100/µg/ml pendant 24h, mais également en présence ou en absence de céramides à différentes concentrations (1/20, 1/50 et 1/100).

Ensuite les kératinocytes seront récupérés et le niveau d'expression de la protéine de jonction E-cadhérine ainsi que de la métalloprotéinase MMP-9 ont été déterminés par immunofluorescence et western blot. Toutes ces expériences ont été réalisées à trois reprises.

Les résultats ont confirmé la diminution du niveau d'expression de la e-cadhérine en présence des extraits d'acariens de la poussière (HDM). Les résultats ont également montré que les céramides DS et les céramides Y30 n'ont montré aucun effet sur la diminution de ce niveau d'expression de la e-Cadhérine en présence d'extraits d'acariens de la poussière (HDM).

En revanche et de façon inattendue, les céramides HP (SEPPIC) ont montré une inhibition claire de l'effet de HDM sur le niveau d'expression de la e-Cadhérine. En effet, en présence de HDM et des 3 concentrations de céramides HP, le niveau de E-cadhérine est resté stable.

La détermination du mécanisme impliqué dans ces résultats a permis de révéler que HDM augmente le niveau d'expression de la métalloprotéase MMP9, laquelle augmentation est inhibée par les céramosides HP. Il apparaît donc que la combinaison de céramides et de digalactosyldiacylglycérides (DGDG) présents au sein de cet actif (correspondant à de l'huile extraite de farine de blé) permette d'obtenir cet effet surprenant permettant le maintien de jonctions serrées normales en présence de HDM.

Des résultats similaires ont été obtenus en utilisant d'autres sources d'extrait de lipides polaires de germe de blé (WHEAT CERASOMES OIL, EPI) ainsi qu'avec de l'huile de germe de blé.

Les résultats ont également montré que l'adjonction d'AD-Résyl permettait de potentialiser l'efficacité du céramide HP.

### Exemple 2 : Efficacité de la composition selon l'invention... :

Des biopsies de peau de 4 mm de diamètre issues de 3 volontaires sains ont été prélevés.

Ces biopsies ont été incubées en présence ou en absence d'extraits d'acariens de la poussière (HDM) à 100/µg/ml pendant 30 minutes. La biopsie a été ensuite recouverte d'une crème comprenant 0,08 ou 0,1% de céramide HP et remise en culture pendant 24h en présence ou en absence de HDM.

Les résultats ont montré qu'aux deux concentrations, les céramides HP ont permis de préserver l'intégrité structurale de la peau à la différence des contrôles.

## Revendications

1. Une composition dermatologique comprenant au moins un extrait à base de lipides polaires de germe de blé pour une utilisation pour prévenir et/ou traiter une dermatite atopique consécutive d'une réaction allergique aux acariens de poussière chez un sujet.

2. La composition dermatologique pour utilisation selon la revendication 1, **caractérisée en ce qu'**elle vise à prévenir et/ou traiter l'altération des j onctions serrées associée à une dermatite atopique consécutive d'une réaction allergique aux acariens de poussière chez un sujet.

3. La composition dermatologique pour utilisation selon la revendication 1, **caractérisée en ce que** cette dermatite atopique est consécutive d'une réaction allergique à la peptidase 1 (Der p1 ou Der f1) des acariens de poussière.

4. La composition dermatologique pour utilisation selon l'une quelconque des revendications précédentes, **caractérisée en ce que** le sujet est un humain.

5. La composition dermatologique pour utilisation selon l'une quelconque des revendications précédentes, **caractérisée en ce que** ledit extrait est sous forme d'une huile, d'une émulsion, ou d'une poudre.

6. La composition dermatologique pour utilisation selon la revendication précédente, **caractérisée en ce que** ledit extrait est sous forme d'une poudre.

7. La composition dermatologique pour utilisation selon la revendication précédente, **caractérisée en ce que** ledit extrait présente une teneur en céramides de 40% ou plus (en poids par rapport au poids total de l'huile), de préférence de 50% ou plus

8. La composition dermatologique pour utilisation selon la revendication 6, **caractérisée en ce que** ledit extrait é présente une teneur en digalactosyldiacylglycérides de 30% ou plus (en poids par rapport au poids total de l'huile), de préférence de 40% ou plus.

9. La composition dermatologique pour utilisation selon l'une quelconque des revendications précédentes, **caractérisée en ce que** la teneur de la composition selon l'invention extrait est comprise entre 0,01 et15% (en poids par rapport au poids total de la composition).

10. La composition dermatologique pour utilisation selon l'une quelconque des revendications précédentes, **caractérisée en ce qu'**elle comprend en outre des oligofructanes d' *Ophiopogon japonicus.*

11. La composition pour utilisation selon la revendication précédente, **caractérisée en ce que** les oligofructanes d*'Ophiopogon japonicus* sont les polysaccharides présents dans ses tubercules.

12. La composition pour utilisation selon l'une quelconque des revendications 10 ou 11, **caractérisée en ce que** la teneur en oligofructanes d*' Ophiopogon japonicus* est comprise entre 0,01 et 5% (en poids par rapport au poids total de la composition), de préférence elle est comprise entre 0,05 et 1% et, de manière particulièrement préférée, entre 0,3 et 0,7%.

## Patentansprüche

1. Dermatologische Zusammensetzung, die mindestens einen Extrakt auf Basis von polaren Weizenkeimlipiden umfasst, für eine Verwendung zum Vorbeugen und/oder Behandeln einer atopischen Dermatitis infolge einer allergischen Reaktion gegenüber Staubmilben bei einem Subjekt.

2. Dermatologische Zusammensetzung zur Verwendung nach Anspruch 1, **dadurch gekennzeichnet, dass** sie darauf abzielt, die Änderung der engen Bindungen in Verbindung mit einer atopischen Dermatitis infolge einer allergischen Reaktion gegenüber Staubmilben bei einem Subjekt vorzubeugen und/oder zu behandeln.

3. Dermatologische Zusammensetzung zur Verwendung nach Anspruch 1, **dadurch gekennzeichnet, dass** diese atopische Dermatitis auf eine allergische Reaktion gegenüber der Peptidase 1 (Der p1 oder Der f1) der Staubmilben folgt.

4. Dermatologische Zusammensetzung zur Verwendung nach einem der vorstehenden Ansprüche, **dadurch gekennzeichnet, dass** das Subjekt ein Mensch ist.

5. Dermatologische Zusammensetzung zur Verwendung nach einem der vorstehenden Ansprüche, **dadurch gekennzeichnet, dass** der Extrakt in Form eines Öls, einer Emulsion oder eines Pulvers vorliegt.

6. Dermatologische Zusammensetzung zur Verwendung nach dem vorstehenden Anspruch, **dadurch gekennzeichnet, dass** der Extrakt in Form eines Pulvers vorliegt.

7. Dermatologische Zusammensetzung zur Verwendung nach dem vorstehenden Anspruch, **dadurch gekennzeichnet, dass** der Extrakt einen Gehalt an Ceramiden von 40% oder mehr (Gewichts-% in Bezug auf das Gesamtgewicht des Öls), vorzugsweise 50% oder mehr aufweist.

8. Dermatologische Zusammensetzung zur Verwendung nach Anspruch 6, **dadurch gekennzeichnet, dass** der Extrakt é einen Gehalt an Digalactosyldiacylglyceriden von 30% oder mehr (Gewichts-% in Bezug auf das Gesamtgewicht des Öls), vorzugsweise 40% oder mehr aufweist.

9. Dermatologische Zusammensetzung zur Verwendung nach einem der vorstehenden Ansprüche, **dadurch gekennzeichnet, dass** der Gehalt des Extrakts der Zusammensetzung gemäß der Erfindung zwischen 0,01 und 15% (Gewichts-% in Bezug auf das Gesamtgewicht der Zusammensetzung) liegt.

10. Dermatologische Zusammensetzung zur Verwendung nach einem der vorstehenden Ansprüche, **dadurch gekennzeichnet, dass** sie weiter Oligofruktane von *Ophiopogon japonicus* umfasst.

11. Dermatologische Zusammensetzung zur Verwendung nach dem vorstehenden Anspruch, **dadurch gekennzeichnet, dass** die Oligofruktane von *Ophiopogon japonicus* Polysaccharide sind, die in seinen Knollen vorhanden sind.

12. Dermatologische Zusammensetzung zur Verwendung nach einem der Ansprüche 10 oder 11, **dadurch gekennzeichnet, dass** der Gehalt an Oligofruktanen von *Ophiopogon japonicus* zwischen 0,01 und 5% (Gewichts-% in Bezug auf das Gesamtgewicht der Zusammensetzung) liegt, er vorzugsweise zwischen 0,05 und 1% und besonders bevorzugt zwischen 0,3 und 0,7% liegt.

## Claims

1. A dermatological composition comprising at least one extract based on polar lipids of wheat germ for use in preventing and/or treating atopic dermatitis resulting from an allergic reaction to dust mites in a subject.

2. The dermatological composition for use according to claim 1, **characterized in that** it aims to prevent and/or treat the alteration of tight junctions associated with atopic dermatitis resulting from an allergic reaction to dust mites in a subject.

3. The dermatological composition for use according to claim 1, **characterized in that** this atopic dermatitis results from an allergic reaction to peptidase 1 (Der p1 or Der f1) of dust mites.

4. The dermatological composition for use according to any one of the preceding claims, **characterized in that** the subject is a human.

5. The dermatological composition for use according to any one of the preceding claims, **characterized in that** said extract is in the form of an oil, an emulsion, or a powder.

6. The dermatological composition for use according to the preceding claim, **characterized in that** said extract is in the form of a powder.

7. The dermatological composition for use according to the preceding claim, **characterized in that** said extract has a ceramide content of 40% or more (by weight relative to the total weight of the oil), preferably of 50% or more.

8. The dermatological composition for use according to claim 6, **characterized in that** said extract has a digalactosyldiacylglycerides content of 30% or more (by weight relative to the total weight of the oil), preferably of 40% or more.

9. The dermatological composition for use according to any one of the preceding claims, **characterized in that** the extract content of the composition according to the invention is comprised between 0.01 and 15% (by weight relative to the total weight of the composition).

10. The dermatological composition for use according to any one of the preceding claims, **characterized in that** it further comprises oligofructans of *Ophiopogon japonicus.*

11. The composition for use according to the preceding claim, **characterized in that** the oligofructans of *Ophiopogon japonicus* are the polysaccharides present in its tubers.

12. The composition for use according to any one of claims 10 or 11, **characterized in that** the content of oligofructans of *Ophiopogon japonicus* is comprised between 0.01 and 5% (by weight relative to the total weight of the composition), preferably it is comprised between 0.05 and 1% and, particularly preferably, between 0.3 and 0.7%.
